(19) Europäisches Patentamt
European Patent Office
Office européen des brevets

(11) EP 2 123 632 B1

(12) **EUROPEAN PATENT SPECIFICATION**

(45) Date of publication and mention of the grant of the patent:
**27.06.2012 Bulletin 2012/26**

(21) Application number: **08711704.0**

(22) Date of filing: **14.02.2008**

(51) Int Cl.:
***C07C 249/16*** (2006.01) ***C07C 251/88*** (2006.01)
***B01J 19/00*** (2006.01) ***C01B 21/16*** (2006.01)

(86) International application number:
**PCT/JP2008/052915**

(87) International publication number:
**WO 2008/102830 (28.08.2008 Gazette 2008/35)**

# (54) METHOD FOR PRODUCING KETAZINE COMPOUND

VERFAHREN ZUR HERSTELLUNG EINER KETAZINVERBINDUNG

PROCÉDÉ DE PRODUCTION D'UN COMPOSÉ DE CÉTAZINE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **20.02.2007 JP 2007039185**

(43) Date of publication of application:
**25.11.2009 Bulletin 2009/48**

(73) Proprietor: **Otsuka Chemical Co., Ltd.**
**Osaka-shi,**
**Osaka 540-0021 (JP)**

(72) Inventors:
- **TANIGUCHI, Masatoshi**
  **Osaka-shi**
  **Osaka 540-0021 (JP)**
- **MORI, Koji**
  **Tokushima-shi**
  **Tokushima 771-0193 (JP)**

(74) Representative: **Winter, Brandl, Fürniss, Hübner, Röss, Kaiser, Polte - Partnerschaft**
**Alois-Steinecker-Strasse 22**
**85354 Freising (DE)**

(56) References cited:
**EP-A1- 0 115 076 JP-A- 2004 067 633**

- **"HYDRAZINE AND ITS DERIVATIVES ED - MARK H F; OTHMER D F; OVERBERGER C G; SEABORG G T" 1 January 1989 (1989-01-01), KIRK - OTHMER ENCYCLOPEDIA OF CHEMICAL TECHNOLOGY. GRAVITY CONCENTRATION TO HYDROGEN ENERGY, NEW YORK, WILEY & SONS, US, PAGE (S) 734 - 755 , XP002032138 * pages 749-755 ***
- **KAUR RAMINDER ET AL: "Agitation Effects in a Gas-Liquid-Liquid Reactor System: Methyl Ethyl Ketazine Production" INTERNATIONAL JOURNAL OF CHEMICAL REACTOR ENGINEERING, BERKELEY ELECTRONIC PRESS, BERKELEY, CA, US, vol. 5, 1 January 2007 (2007-01-01), pages A27/1-19, XP009134382 ISSN: 1542-6580**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 123 632 B1

## Description

TECHNICAL FIELD

The present invention relates to a process for preparing ketazine compounds.

BACKGROUND ART

Ketazine is useful as an intermediate obtained in the course of the preparation of hydrazine hydrate. It is known that ketazine can be prepared from hydrogen peroxide, ammonia and ketone (Nonpatent Literatures 1 and 2). The hydrazine produced in the course of the reactions involved reacts with the remaining oxidizing agent, whereby the hydrazine is decomposed to entail the likelihood of lowering the yield of the desired ketazine product. Furthermore, ketone, amine and the hydrazine produced give rise to a complex side reaction, producing high-boiling organic by-products which are represented, for example, by the formula (A) or (B) and which are difficult to remove

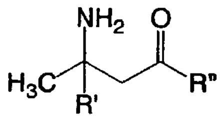
(A),
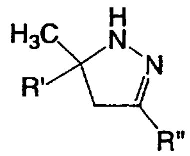
(B)

wherein R' and R" are the same or different and are each methyl or ethyl.

Accordingly, the ketone and ammonia to be used are actually used in large excess to conduct the reaction so as to promptly consume the oxidizing agent to be used. However, the use of large amounts of ketone and ammonia greatly decreases the concentration of ketazine in the reaction mixture to a level which is usually as low as up to 3%.

[Nonpatent Literature 1] Kirk-Othmer 3rd Ed., Vol. 12, pp. 734-755.
[Nonpatent Literature 2] Toshio Yokota "Hydrazine, Properties and Application thereof," Chijin Shokan, March 1968. Hydrazine hydrate is prepared from the ketazine obtained generally by distilling under pressure an aqueous solution of ketazine produced.

However, if the ketazine concentration of the reaction mixture is low, a problem arises in that the pressure distillation for effecting a reaction for affording hydrazine hydrate requires a large quantity of energy.

When the reaction is so conducted as to give a high concentration of ketazine to avoid this problem, by-products become mixed as impurities with the hydrazine hydrate to be produced, in addition to the reduced ketazine yield mentioned.

In EP 0 115 076 is disclosed a process for producing azine compounds by reacting a carbonyl compound with ammonia and calcium hypochlorite. A high yield of the azine compound could be achieved by carrying out the reaction under specific conditions of a considerably lower feed rate of the calcium hypochlorite to the reaction system than described in the prior art. The reaction is preferably carried out in a continuous vessel-type reactor.

Kaur, R. et al., "International Journal of Chemical Reactor Engineering", Volume 5, 2007, p. A27/1-19, used the formation of methylethylkeatazine as a typical case of a homogeneous catalyzed gas-liquid-liquid reaction to study agitation effects on the reaction in a semi-batch reactor. They found out that the yield of ketazine improved with degree of agitation and that a vaned disk stirrer is more effective compared to a flat paddle stirrer.

An object of the present invention is to provide a process for preparing ketazine compounds which is capable of giving the ketazine compound in a high yield with by-products inhibited, the process being capable of affording a reaction mixture of ketazine compound in a high concentration.

DISCLOSURE OF THE INVENTION

The present invention provides the following.

1. A process for preparing a ketazine compound of the formula (1) from a ketone compound of the formula (2), ammonia and an oxidizing agent, wherein a solution containing the ketone compound of the formula (2) and ammonia is brought into contact with an aqueous solution of the oxidizing agent in a tubular reactor having a flow channel width of 2 to 10000 μm

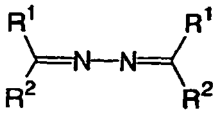

(1)

wherein $R^1$ and $R^2$ are the same or different and are each a $C_{1-6}$ alkyl group, or $R^1$ and $R^2$ are combined with each other into a straight-chain $C_{2-7}$ alkylene group

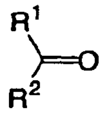

(2)

wherein $R^1$ and $R^2$ are the same as above, and
wherein the oxidizing agent is hydrogen peroxide or sodium hypochlorite.
2. A process as defined above wherein 2 to 5 moles of the ketone compound of the formula (2) and 2 to 10 moles of ammonia are used per mole of the hydrogen peroxide.
3. A process as defined above wherein 2 to 50 moles of the ketone compound of the formula (2) and 2 to 400 moles of ammonia are used per mole of effective chlorine of the sodium hypochlorite.
4. A process as defined above wherein the mixture to be reacted and comprising the solution containing the ketone compound of the formula (2) and ammonia and the aqueous solution of the oxidizing agent contains the oxidizing agent in an amount of 1.6 to 20 wt. %.

We have carried out intensive research to fulfill the foregoing object and found that when a reaction is conducted in a tubular reactor having a very small flow channel width for preparing ketazine from ketone, ammonia and hydrogen peroxide or sodium hypochlorite as an oxidizing agent, a ketazine compound can be produced in a high yield with the formation of by-products inhibited even if the reaction mixture contains the ketazine compound in a high concentration. This, the present invention has been accomplished.

The substituents herein mentioned mean the following. Examples of $C_{1-6}$ alkyl groups are straight-chain or branched-chain alkyl groups having 1 to 6 carbon atoms, such as methyl, ethyl, n-propyl, isopropyl, n-butyl, isobutyl, sec-butyl, tert-butyl, n-pentyl, isopentyl, neopentyl, tert-pentyl, n-hexyl and isohexyl.

Examples of straight-chain $C_{2-7}$ alkylene groups are ethylene, trimethylene, tetramethylene, pentamethylene, hexamethylene and heptamethylene.

According to the invention, a solution containing a ketone compound of the formula (2) and ammonia is brought into contact with an aqueous solution containing hydrogen peroxide or sodium hypochlorite as an oxidizing agent in a tubular reactor, whereby a ketazine compound of the formula (1) is prepared.

Examples of ketone compounds of the formula (2) for use in the preparation process of the present invention are acetone, 2-pentanone, 3-pentanone, methyl ethyl ketone, methyl isopropyl ketone, methyl isobutyl ketone, cyclobutanone, cyclopentanone and cyclohexanone. Especially preferable are acetone, methyl ethyl ketone and methyl isobutyl ketone.

The ammonia to be used in the process of the invention may be ammonia water commercially available, but it is preferable to prepare ammonia water at any desired high concentration by introducing ammonia gas into water. The solution containing a ketone compound of the formula (2) and ammonia is an aqueous solution obtained by dissolving the ketone compound of the formula (2) and ammonia in water. The solution may be prepared, for example, by mixing the ketone compound of the formula (2) with ammonia water and diluting the resulting solution to a predetermined concentration as required. Alternatively, the solution can be prepared by mixing an aqueous solution of the compound of the formula (2) with ammonia water.

An aqueous solution containing the ketone compound of the formula (2) and ammonia and prepared in advance may be introduced into a tubular reactor for reaction, or an aqueous solution of the ketone compound of the formula (2) and ammonia water may be admitted into the tubular reactor through respective inlets and brought into contact with each other in the flow channel.

It is thought that in the solution containing the ketone compound of the formula (2) and ammonia, these compounds react partly or wholly to form a ketimine compound of the formula (3) given below

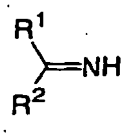

(3)

wherein $R^1$ and $R^2$ are the same as above.

One mole of the ketimine compound corresponds to 1 mole of the ketone compound of the formula (2) and 1 mole of ammonia.

The oxidizing agents for use in the process of the invention are sodium hypochlorite and hydrogen peroxide.

The hydrogen peroxide can be a commercial product. A 30 to 90 wt. % aqueous solution of hydrogen peroxide is usually usable. Such hydrogen peroxide may contain phosphoric acid or like stabilizer which is usually used for hydrogen peroxide.

The sodium hypochlorite to be used is an aqueous solution of sodium hypochlorite commercially available and 10 to 20 wt. % in effective chlorine concentration, or desalted sodium hypochlorite which is reduced in sodium chloride content that is produced as a by-product depending on the production apparatus or conditions.

In the case where hydrogen peroxide is used as the oxidizing agent, the ketone compound of the formula (2), ammonia and oxidizing agent are used, for example, in such amounts that 2 to 5 moles, preferably about 3 to about 4 moles, of the ketone compound of the formula (2) and 2 to 10 moles, preferably about 3 to about 4 moles, of ammonia are used per mole of hydrogen peroxide.

In the case where sodium hypochlorite is used as the oxidizing agent, 2 to 50 moles, preferably about 4 to about 40 moles, of the ketone compound of the formula (2) and 2 to 400 moles, preferably about 3 to about 300 moles, of ammonia are used per mole of effective chlorine of the sodium hypochlorite.

In the preparation process of the invention, the oxidizing agent is used at a concentration which can be determined over a wide range. In the mixture to be reacted and comprising a solution containing the ketone compound of the formula (2) and ammonia and an aqueous solution containing the oxidizing agent, the oxidizing agent is used at a concentration of 0.1 to 30 wt. %, preferably 1.6 to 20 wt. %, more preferably 2 to 15 wt. %.

In preparing ketazine, it is generally thought preferable to practice the process so that the mixture to be reacted will be up to 3 wt. % in the concentration of hydrazine or ketazine so as to control the reaction with hydrazine in the reaction process. It therefore follows that the concentration of the oxidizing agent should be limited to about 1 wt. % in the case where hydrogen peroxide is used.

The process of the invention affords the ketazine compound of the formula (1) without permitting the concentration of the oxidizing agent in the mixture to be reacted to influence the yield regardless of whether the concentration is low or high. For example in the case where 2 wt. % of the oxidizing agent is used, the reaction mixture affords theoretically about 6.6 wt. % of ketazine compound.

When hydrogen peroxide is used as the oxidizing agent, it is desirable to use a catalytically active compound. Preferable to use are, for example, an amide compound, ammonium salt or nitrile compound disclosed in JP2004-67633A, and an operating fluid which is prepared by dissolving an organic arsenic compound and carboxylic acid in a solvent mixture of water and an alcohol.

Preferably, the operating fluid is brought into contact or mixed with the solution containing a ketone compound of the formula (2) and ammonia, before being brought into contact or mixed with the aqueous solution containing the oxidizing agent.

Alternatively, the operating fluid may have added thereto a portion of the ketone compound of the formula (2) or ammonia to be used.

When the operating fluid is used, the concentration of the oxidizing agent for use in the mixture to be reacted is the concentration in the operating fluid and the mixture which comprises the solution containing the ketone compound of the formula (2) and ammonia and the aqueous solution containing the oxidizing agent.

The reaction can be conducted usually at 30 to 110° C, preferably 30 to 70° C. If the reaction temperature is lower than 30° C, a lower reaction yield will result, whereas if the temperature is higher than 110° C, hydrogen peroxide or sodium hypochlorite will decompose to similarly result in a lower reaction yield. Although the reaction pressure is optional, the reaction is easy to conduct at atmospheric pressure.

The ketazine compound of the formula (1) obtained by the present reaction can be isolated from the reaction mixture by a known method, such as liquid-liquid separation using a mixer/settler or centrifuge, liquid-liquid extraction or distillation, or a combination of such methods.

The tubular reactor for use in the present invention has a liquid inlet, a liquid outlet and a flow channel for causing a liquid to flow from the inlet to the outlet. Preferably the reactor has two or three inlets for admitting different fluids through the respective inlets. After coming into contact with each other or one another, the fluids are run off from the outlet. The flow channel can be branched to a T shape or Y shape. The flow channel portion for bringing the fluids

into contact with one another may hereinafter be referred to as a "reaction channel."

Such a tubular reactor can be made by forming a flow channel in the surface of a substrate by various methods including lamination, affixing, etching, LIGA (Lithographie, Galvanoformung, Abformung) process, cutting and molding. It is also possible to use commercial reactors such as Microfluidics chips manufactured by Arbiotec, Ltd., microreactors manufactured by Institute fur Mikrotechnik Mainz GmbH, Selecto or Cytos manufactured by Cellular Process Chemistry GmbH, etc.

The flow channel is not particularly limited in cross sectional shape. For example, the channel may be triangular, square, rectangular, pentagonal, hexagonal, octagonal or otherwise polygonal, circular or elliptical in cross section.

The flow channel may be smooth-surfaced or may have minute projections or indentations or a helix provided by such projections or indentations when so desired.

The flow channel is 2 to 10000 μm, preferably 5 to 5000 μm, in width. If the flow channel width is smaller than 2 μm, the channel becomes more likely to be clogged up with solids separating out, whereas if the width is greater than 10000 μm, it become difficult to obtain the effect contemplated by the present invention. Incidentally, the term "flow channel width" refers to the greatest width of the flow channel portion where at least two fluids can be brought into contact with each other. When the flow channel is circular in cross section, this width corresponds to the diameter.

The channel, i.e., the reaction channel, is about 0.01 to about 100 m, preferably about 0.05 to about 10 m, in length. The channel may be in the form of a straight or bent tube or a circular, elliptical or square to rectangular coil, or may have a helical shape.

The process of the invention will be described briefly with reference to the tubular reactor shown in FIG. 1. An aqueous solution containing the ketone compound of the formula (2) is admitted through an inlet (a) 2, and an aqueous solution containing ammonia introduced through an inlet (b) 3, whereby the solutions are mixed together within a flow channel 5a to provide a solution containing the ketone compound of the formula (2) and ammonia. An aqueous solution containing an oxidizing agent is introduced through an inlet (c) 4. The solution containing the ketone compound of the formula (2) and ammonia can be brought into contact with and mixed with the aqueous solution containing the oxidizing agent in the portion of the flow channel downstream from the inlet 4. The resulting ketazine compound of the formula (1) produced can be delivered from an outlet 6 along with the reaction mixture.

In the case where hydrogen peroxide is used, a ketazine compound of the formula (1) can be prepared by admitting through the inlet (a) 2 an aqueous solution prepared by mixing an aqueous solution containing a ketone compound of the formula (2) with an aqueous solution of ammonia in advance, introducing an operating fluid through the inlet (b)3 for contact or mixing in the channel (5a) to form a solution containing the ketone compound of the formula (2) and ammonia, and admitting an aqueous solution (aqueous solution of hydrogen peroxide) containing the oxidizing agent through the inlet (c)4.

It is desired that the tubular reactor for use in the process of the invention be so designed that when at least two fluids admitted through the inlets are brought into contact or mixed with each other and flow through the reaction channel, each of the fluids will be in the form of a laminar flow.

This state of laminar flow is represented by a Reynolds number of the following Equation (1). This number is preferably smaller than 2300, more preferably up to 100

$$Re = LU\rho / \eta \qquad (1)$$

wherein Re is Reynolds number, L is the length of the flow channel, U is the flow velocity of the fluid, ρ is the density of the fluid and n is the coefficient of viscosity of the fluid.

With the reactor having a minute structure for use in the present invention, the smaller the variations in the pressure of the fluid flowing through the reactor, the higher the contact or mixing efficiency. This suppresses the rise in local temperatures, inhibiting side reactions and resulting in a higher reaction efficiency.

The pressure variations should be up to 5%, preferably up to 2%, more preferably up to 1%.

If the pressure variations are not smaller than 5%, the flow rate and flow velocity vary with the variations, with the result that the reactor fails to maintain a uniform contact or mixing ratio to entail a lower reaction efficiency.

The method of admitting the fluids is not particularly limited insofar as the method can introduce the fluid at a stabilized flow rate (flow velocity) and stabilized pressure and is capable of forming a stabilized laminar flow. Preferable to use are pumps such as a syringe pump, piston pump, diaphragm pump, plunger pump, gear pump, peristaltic pump, volute pump and diffuser pump. These pumps can be used singly, or at least two kinds of them are usable in combination.

The tubular reactor for use in the present invention can be used with suitably selected related devices attached thereto in conformity with the reaction conditions. These devices include, for example, a cooling device, heater, electric controller and analyzer.

When a plurality of tubular reactors of the type described are used as arranged in parallel or in superposed

layers, industrial quantity production becomes feasible.

BRIEF DESCRIPTION OF THE DRAWINGS

FIG. 1 is a diagram shown an example of tubular reactor for use in the process of the invention; and
FIG. 2 is a diagram showing an example of tubular reactor used in Examples of the invention.
1: substrate, 2: inlet (a), 3: inlet (b), 4: inlet (c), 5: flow channel, 5a: portion of the flow channel. 6: outlet.

BEST MODE OF CARRYING OUT THE INVENTION

The present invention will be described below in detail with reference to Reference Examples, Examples and Comparative Examples.

Reference Example 1 (Fabrication of Tubular Reactor)

A tubular reactor was fabricated under the following conditions using a cutting machine (having a small machining center EGX-300, product of Roland) for a soda-lime glass sheet, measuring 30 mm × 70 mm × 2 mm.

Cutting drill: electrodeposited diamond bars (A2505 and A2502. products of MINIMO Co., Ltd.) measuring 0.5 mm and 0.2 mm in diameter
Cutting conditions: speed in the directions of XY-axes 0.5 mm/sec, speed in the direction of Z-axis 0.5 mm/sec, cutting pitch 0.01 mm, main shaft revolution number 15000 rpm.
Inlets and outlet: machined by using a solid carbide drill (A2501), 1.00 mm in diameter.

An unmachined glass sheet was superposed on the machined soda-lime glass sheet obtained and fused thereto at 660° C for at least 5 hours to make a reactor (FIG. 2) having a flow channel 100 to 500 μm in width, 100 to 200 μm in depth and 150 mm [(a)-(b): 50 mm, (b)-outlet: 100 mm] in length.
The reactor was equipped with a thermostat, and each of the inlets (inlets A-C) was provided with a syringe pump (KD, product of Scientific Inc.)

Example 1

An operating fluid (100 g) was prepared by mixing 33 wt. % of cacodylic acid, 7 wt. % of methylarsonic acid, 20 wt. % of acetic acid, 7 wt. % of water, 6 wt. % of ammonia and 27 wt. % of ethylene glycol.
With the thermostat set at 50° C for the tubular reactor fabricated in Reference Example 1, the syringe pumps were used for admitting the operating fluid into the reactor through the inlet A, an aqueous solution of 24 g of 23% ammonia water and 46 g of methyl ethyl ketone through the inlet B and 17 g of 60% aqueous solution of hydrogen peroxide through the inlet C. These solutions were so introduced as to be mixed together in the ratio of 10:7:2. The flow rate of the reaction mixture (at (b) to the outlet in FIG. 2) was 4.5 ml/min. The Reynolds number at this time was about 2.
An excess of methyl ethyl ketone was added to the reaction mixture run off from the outlet, and the mixture was subjected to high performance liquid chromatography (HPLC) under the following conditions. The amount of methyl ethyl ketazine produced was measured by comparison with an authentic product.

[HPLC Conditions]

Column: Inertsil ODS-3, (diam.4.6 × 250 mm, product of GL Science)
Column temp. : 40° C
Mobile phase: acetonitrile/20 mmoles phosphoric acid buffer (pH 7) = 2/8
Flow velocity: 1.0 ml/min.
Detector: UV (λ 235 nm)
Amount of injection: 3 μl

Consequently, the yield of the desired product, methyl ethyl ketazine, was found to be 95% based on the hydrogen peroxide used.

Example 2

The syringe pumps were used for introducing 813 g of 23% aqueous solution of ammonia into the tubular reactor fabricated in Reference Example 1 through the inlet A, 174 g of acetone through the inlet B and 253 g of aqueous solution of sodium hypochlorite which was 17% in effective chlorine content through the inlet C. These solutions were admitted so as to be mixed together in the ratio of 81:17:25. The flow rate of the reaction mixture (at (b) to the outlet in FIG. 2) was 1.5 ml/min. The Reynolds number at this time was about 1.5.

The reaction mixture run off from the outlet was analyzed by the following chromatographic procedure, and the amount of dimethyl ketazine was determined with reference to the calibration curve with use of an authentic product.

[Conditions for Chromatographic Analysis]

Column: PEG 20M + KOH (10 + 10%) on Chromosorb W N
AW 80/100 mesh 2.1 m
Column temp.: 170° C
Injection temp.: 230° C
Carrier gas: $N_2$ 40 ml/min
Detector: FID
Amount of injection: 0.5 μl

As a result, the yield of the desired product, dimethyl ketazine, was found to be 96% based on the effective chlorine content of the sodium hypochlorite used.

Reference Example 2

The reaction mixture containing methyl ethyl ketazine and obtained in Example 1 was distilled under elevated pressure (internal temperature: up to 150° C, 2229 hPa), and the time point when the methyl ethyl ketone content in the resulting fraction reached a level no longer detectable or lower was taken as the termination point (gas chromatographic analysis).

The residue obtained was further distilled at a reduced pressure of 160 hPa and thereby adjusted so as to become an 80% aqueous solution of hydrazine hydrate. This 80% aqueous solution of hydrazine hydrate was cooled to room temperature and thereafter filtered to separate insolubles and precipitate off to obtain the desired 80% aqueous solution of hydrazine hydrate (yield: 96% from ketazine).

The hydrazine hydrate content was calculated by the following titration method.

[Hydrazine Hydrate Content]

A 10 ml quantity of sample was accurately measured out using a whole pipette and placed into a volumetric flask (100 mL), and deionized water was further placed in to a predetermined amount. A 10 ml quantity of the resulting mixture was then transferred to an Erlenmeyer flask using a whole pipette. Subsequently, about 90 ml of deionized water and about 5 ml of sulfuric acid (1 + 1)[which was concentrated sulfuric acid as diluted with the same volume of water] were further placed in, and the resulting mixture was boiled for evaporation until the quantity of the mixture reduced to one half. A small excess of sodium hydrogencarbonate was added to the resulting mixture as cooled (to such an extent that some crystals remained), and the mixture was titrated with one-tenth normal iodine. Starch was used as an indicator. The hydrazine hydrate content (w/v %) was calculated from the following equation.

$$\text{Hydrazine hydrate content (w/v \%)} = 100 \times (0.00125 \times 100 \times F \times A/10 \times 10) = 0.125 \times F \times A$$

A: amount of 1/10N iodine used for titration (ml)
F: the titer of 1/10N iodine = value of iodine measured out/25.3809 (g) as dissolved in 1000 ml of deionized water.

Reference Example 3

By the same method as in Reference Example 2, an 80% aqueous solution of hydrazine hydrate was prepared from the reaction mixture obtained in Example 2 and containing dimethyl ketazine (yield: 95% from ketazine).

Comparative Example 1

Into a 200-cc reactor (four-necked flask) equipped with a stirrer was placed 100 g of an operating fluid comprising 33 wt. % of cacodylic acid, 7 wt. % of methylarsonic acid, 20 wt. % of acetic acid, 7 wt. % of water, 6 wt. % of ammonia and 27 wt. % of ethylene glycol. The operating fluid was maintained at 60° C, and 24 g of 23% aqueous solution of ammonia, 46 g of methyl ethyl ketone and 8.5 g of 60% aqueous solution of hydrogen peroxide were added to the liquid at the same time over a period of 30 minutes, followed by a reaction for 80 minutes. The reaction mixture obtained was analyzed under the HPLC conditions given in Example 1 to find that methyl ethyl ketazine was produced in a yield of 67% based on the hydrogen peroxide used.

An 80% aqueous solution of hydrazine hydrate was further obtained in the same manner as in Reference Example 2 (yield: 89% from ketazine). By-products A and B were found present in amounts of 205 ppm and 101 ppm, respectively.

Comparative Example 2

A 813 g quantity of 23% aqueous solution of ammonia and 174 g of acetone were placed into a reactor, and 1020 g of water was further placed in to adjust the ammonia and acetone to a concentration of 18 wt. %. The mixture was then heated to a temperature of 60° C with stirring. Subsequently, 253 g of an aqueous solution of sodium hypochlorite, 14% in effective chlorine content, was added to the mixture over a period of 80 minutes for a reaction.

The reaction mixture obtained was analyzed by the same method as in Example 1 to find that dimethyl ketazine was produced in a yield of 52% based on the sodium hypochlorite used. An 80% aqueous solution of hydrazine hydrate was further obtained in the same manner as in Reference Example 2 (yield: 90% from ketazine). By-products A and B were found present in amounts of 101 ppm and 98 ppm, respectively.

Comparative Example 3

A 81.3 g quantity of 23% aqueous solution of ammonia and 17.4 g of acetone were placed into a reactor, and 1706 g of water was further placed in to adjust the reaction reagents to a concentration of 2%. The mixture was then heated to a temperature of 60° C with stirring. Subsequently, 25.3 g of an aqueous solution of sodium hypochlorite, 14% in effective chlorine content, was added to the mixture over a period of 80 minutes for a reaction.

The reaction mixture obtained was analyzed by the same method as in Example 1 to find that dimethyl ketazine was produced in a yield of 86% based on the sodium hypochlorite used.

An 80% aqueous solution of hydrazine hydrate was further obtained in the same manner as in Reference Example 2 (yield: 92% from ketazine). By-products A and B were found present in amounts of 41 ppm and 35 ppm, respectively.

Table 1 collectively shows the results of Examples 1 and 2, Reference Examples 2 and 3 and Comparative Examples 1 to 3.

Table 1

| | Oxidizing agent | Ketazine compound | | Hydrazine hydrate | | |
|---|---|---|---|---|---|---|
| | **A** | **B** (wt.%) | Yield (%) | **C** (%) | **D** (ppm) | **E** (ppm) |
| Ex.1 | 5.24 | 20.3 | 94 | - | - | - |
| Ref.Ex.2 | - | - | - | 96 (90.2) | 16 | 21 |
| Ex.2 | 7.55 (3.60) | 10.9 | 96 | - | - | - |
| Ref.Ex.3 | - | - | - | 95 (91.2) | 8 | 13 |

(continued)

| | Oxidizing agent | Ketazine compound | | Hydrazine hydrate | | |
|---|---|---|---|---|---|---|
| | **A** | **B** (wt.%) | Yield (%) | **C** (%) | **D** (ppm) | **E** (ppm) |
| Com.Ex.1 | 3.99 | 11.0 | 67 | 89 (59.6) | 205 | 101 |
| Com.Ex.2 | 6.15 (2.93) | 4.9 | 53 | 90 (47.7) | 101 | 98 |
| Com.Ex.3 | 0.46 (0.22) | 0.6 | 86 | 92 (79.1) | 41 | 35 |

**A** : Concentration in mixture to be reacted a)
**B** : Concentration in reaction mixture b)
**C** : Yield c)
**D** : Content of by-product A d)
**E** : Content of by-product B e)

a) The concentration, in the mixture to be reacted, of the oxidizing agent used for reaction. The numerical values in the parentheses for Example 2 and Comparison Examples 2 and 3 are each the concentration of effective chlorine of sodium hypochlorite.
b) The concentration of the ketazine compound in the reaction mixture, as calculated with the yield taken as 100%.
c) The yield of hydrazine hydrate produced from the ketazine compound. The numerical values in the parentheses are yields from the oxidizing agent for the ketazine compound preparation reaction.
d) The content of by-product A present in the hydrazine hydrate produced.
e) The content of by-product B present in the hydrazine hydrate produced.

INDUSTRIAL APPLICABILITY

The process for the invention for preparing ketazine compounds affords the compound without lowering the concentration in the reaction mixture of the ketazine produced and with the formation of by-products suppressed while inhibiting the hydrazine produced in the course of the reaction from being decomposed with an oxidizing agent. The use of the reaction mixture obtained by the process of the invention and containing the ketazine compound reduces the energy needed for the production of hydrazine hydrate, affording hydrazine hydrate in a high yield with impurities diminished.

## Claims

1. A process for preparing a ketazine compound of the formula (1) from a ketone compound of the formula (2), ammonia and an oxidizing agent, wherein a solution containing the ketone compound of the formula (2) and ammonia is brought into contact with an aqueous solution of the oxidizing agent in a tubular reactor having a flow channel width of 2 to 10000 μm

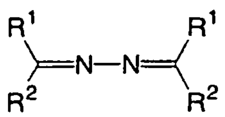

(1)

wherein $R^1$ and $R^2$ are the same or different and are each a $C_{1-6}$ alkyl group, or $R^1$ and $R^2$ are combined with each other into a straight-chain $C_{2-7}$ alkylene group

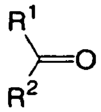

(2)

wherein $R^1$ and $R^2$ are the same as above, and
wherein the oxidizing agent is hydrogen peroxide or sodium hypochlorite.

2. A process as defined in claim 1 wherein 2 to 5 moles of the ketone compound of the formula (2) and 2 to 10 moles of ammonia are used per mole of the hydrogen peroxide.

3. A process as defined in claim 1 wherein 2 to 50 moles of the ketone compound of the formula (2) and 2 to 400 moles of ammonia are used per mole of effective chlorine of the sodium hypochlorite.

4. A process as defined in claim 1 wherein the mixture to be reacted and comprising the solution containing the ketone compound of the formula (2) and ammonia and the aqueous solution of the oxidizing agent contains the oxidizing agent in an amount of 1.6 to 20 wt.%.

**Patentansprüche**

1. Verfahren zur Herstellung einer Ketazinverbindung der Formel (1) aus einer Ketonverbindung der Formel (2), Ammoniak und einem Oxidationsmittel, wobei in einem Rohrreaktor mit einer Strömungskanalbreite von 2 bis 10.000 µm eine Lösung, die die Ketonverbindung der Formel (2) und Ammoniak enthält, in Kontakt gebracht wird mit einer wässrigen Lösung des Oxidationsmittels

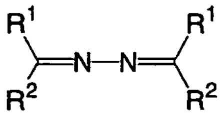

(1)

wobei $R^1$ und $R^2$ gleich oder verschieden sind und jeweils eine $C_{1-6}$-Alkylgruppe sind, oder $R^1$ und $R^2$ miteinander zu einer geradkettigen $C_{2-7}$-Alkylengruppe kombiniert sind

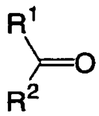

(2)

wobei $R^1$ und $R^2$ wie oben sind, und
wobei das Oxidationsmittel Wasserstoffperoxid oder Natriumhypochlorit ist.

2. Verfahren nach Anspruch 1, wobei 2 bis 5 mol der Ketonverbindung der Formel (2) und 2 bis 10 mol an Ammoniak pro mol an Wasserstoffperoxid verwendet werden.

3. Verfahren nach Anspruch 1, wobei 2 bis 50 mol der Ketonverbindung der Formel (2) und 2 bis 400 mol an Ammoniak pro mol an effektivem Chlor des Natriumhypochlorits verwendet werden.

4. Verfahren nach Anspruch 1, wobei die Mischung, die umgesetzt wird und die die Ketonverbindung der Formel (2) und Ammoniak enthaltende Lösung und die wässrige Lösung des Oxidationsmittels umfasst, das Oxidationsmittel in einer Menge von 1,6 bis 20 Gew.-% enthält.

## Revendications

**1.** Procédé de préparation d'un composé cétazine de formule (1) à partir d'un composé cétone de formule (2), d'ammoniaque et d'un agent oxydant, dans lequel une solution contenant le composé cétone de formule (2) et l'ammoniaque est mise en contact avec une solution aqueuse de l'agent oxydant dans un réacteur tubulaire présentant une largeur de canal d'écoulement de 2 à 10 000 μm

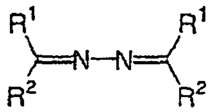
(1)

dans lequel $R^1$ et $R^2$ sont identiques ou différents et sont chacun un groupe alkyle en $C_{1\text{ à }6}$, ou $R^1$ et $R^2$ sont combinés l'un avec l'autre pour former un groupe alkylène en $C_{2\text{ à }7}$ à chaîne droite

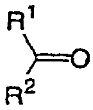
(2)

dans lequel $R^1$ et $R^2$ sont les mêmes que ci-dessus, et
dans lequel l'agent oxydant est le peroxyde d'hydrogène ou l'hypochlorite de sodium.

**2.** Procédé selon la revendication 1, dans lequel 2 à 5 moles du composé cétone de formule (2) et 2 à 10 moles d'ammoniaque sont utilisées par mole du peroxyde d'hydrogène.

**3.** Procédé selon la revendication 1, dans lequel 2 à 50 moles du composé cétone de formule (2) et 2 à 400 moles d'ammoniaque sont utilisées par mole de chlore efficace de l'hypochlorite de sodium.

**4.** Procédé selon la revendication 1, dans lequel le mélange à faire réagir et comprenant la solution contenant le composé cétone de formule (2) et l'ammoniaque et la solution aqueuse de l'agent oxydant contient l'agent oxydant dans une quantité de 1,6 à 20 % en poids.

Fig. 1

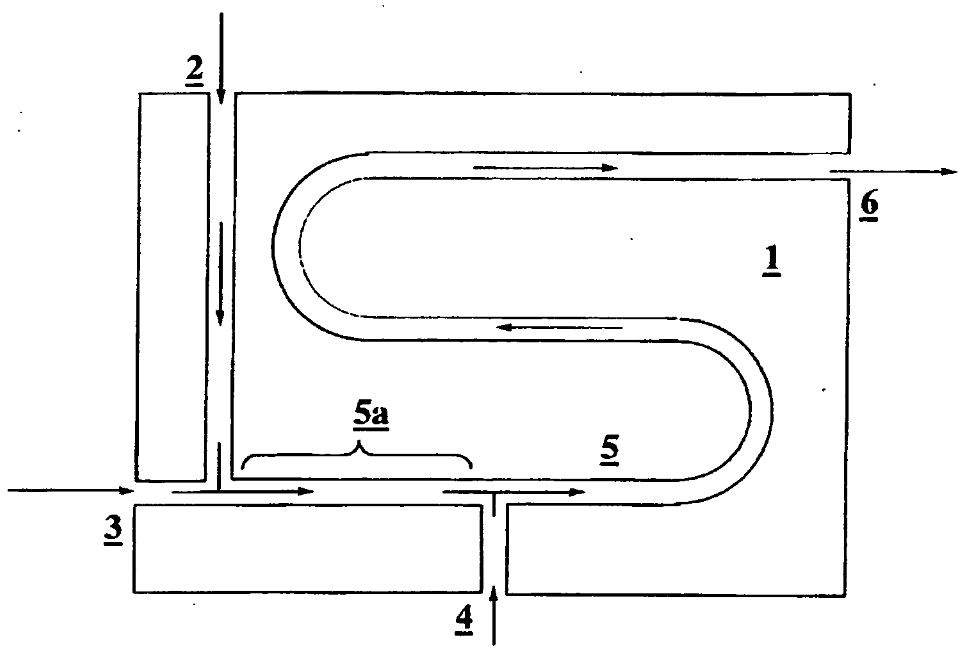


Fig. 2

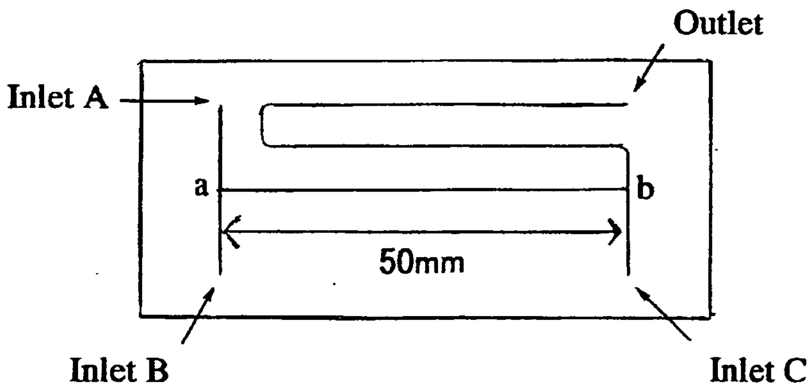

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0115076 A
- JP 2004067633 A


### Non-patent literature cited in the description


- Kirk-Othmer. vol. 12, 734-755
- **Toshio Yokota.** Hydrazine, Properties and Application thereof. Chijin Shokan, March 1968
- **Kaur, R. et al.** *International Journal of Chemical Reactor Engineering,* 2007, vol. 5, A27, 1-19